# EUROPEAN PATENT APPLICATION

(11) **EP 2 266 671 A1**
(43) Date of publication of application: **29.12.2010**
(21) Application number: 08721111.6
(22) Date of filing: 29.02.2008
(51) Int. Cl.: A63B 69/00, A63B 24/00

(54) **MOTION EQUIPMENT SYSTEM**

(71) Applicant: Panasonic Electric Works Co., Ltd, Kadoma-shi Osaka 571-8686 (JP)
(72) Inventor: YOSHIKAWA, Takaaki, Kadoma-shi Osaka 571-8686 (JP); SHINOMIYA, Youichi, Kadoma-shi Osaka 571-8686 (JP); SAKAKIBARA, Hitoshi, Kadoma-shi Osaka 571-8686 (JP)
(74) Representative: Appelt, Christian W.
(86) International application number: PCT/JP2008/053691
(87) International publication number: WO 2009/107242

(57) **Abstract**

Operation time interval measurement means 28 of a passive exercise machine 1 measures the operation time interval and stores the measurement result of the operation time interval in association with the user identifiers in a machine operation table TB2. A server 2 includes an exercise history table TB12 that associates and stores the user identifiers and the measurement data on the operation time interval received by external communication means 41 from the passive exercise machine 1 via a network, operation time interval-to-exercise point conversion means 46 for determining exercise points that are obtained by quantifying the exercise amount of each user from the operation time interval of the user stored in the operation history table TB12 and storing the determined exercise points in the exercise history table TB12, and machine operation pattern determination means 47 for determining a machine operation pattern number indicating an operation state of the passive exercise machine 1 that is being used by the user when a cumulative total number of exercise points per user that has been stored in the exercise history table TB12 exceeds a predetermined threshold level. The server 2 transmits the determined machine operation pattern number from the external communication means 41 to the passive exercise machine 1.

## Description

### TECHNICAL FIELD

The present invention relates to an exercise machine system that promotes the user's health by using an exercise machine such as an automatic exercise machine allowing the user to perform an automatic exercise or a passive exercise machine allowing the user to perform a passive exercise.

### BACKGROUND ART

As disclosed in Japanese Patent Application Laid-open No. 2002-263213, there has been provided an exercise machine system in which a training machine and a server that is connected to the training machine via a network and stores a plurality of kinds of exercise programs to be used in the training machine are connected via a network.

In the aforementioned machine, an exercise program stored in the server can be downloaded to the training machine in response to the user's request, but since the user himself selects the exercise program, an exercise program with an exercise intensity that is higher than the user's exercise capability is sometimes selected, thereby placing a large load on the user's body, or an exercise program with a low exercise intensity is selected and therefore a sufficient exercise effect cannot be obtained.

### DISCLOSURE OF THE INVENTION

The present invention has been created to resolve the above-described problems, and it is an object of the present invention to provide an exercise machine system that can change a load of the exercise machine correspondingly to the exercise level of the user and motivates the user to exercise, thereby promoting the user's health.

In the exercise machine system in accordance with the present invention, one or a plurality of exercise machines for allowing a user to perform an exercise is connected to a server that is operated by a provider of a service that promotes the user's health, via a network.

The exercise machine includes an exercise load application means for applying an exercise load to the user, an operation control means for changing an operation state of the exercise load application means in response to a machine control signal input from the server, identifier storage means for storing identifiers of users that have been allocated to individual users, an operation time interval measurement means for measuring an operation time interval of the exercise machine, a measurement data storage means for storing measurement results of the operation time interval in association with the user identifiers, and a communication means for communicating with the server via the network. The server includes a communication means for communicating with the exercise machine via the network, a server storage means for storing the user identifiers and the measurement data on the operation time interval that have been received by the communication means from the exercise machine in association with each other, a point conversion means for determining exercise points obtained by quantifying an exercise amount of each user from the operation time interval of the user stored in the server storage means and storing the exercise points in the server storage means, and a machine operation control means for transmitting, from the communication means to the exercise machine, a machine control signal for changing an operation state of the exercise machine for the user, when the cumulative total number of exercise points for each user that has been stored in the server storage means exceeds a predetermined threshold.

In accordance with the present invention, the server determines the exercise points on the basis of the operation time interval measured in the exercise machine, transmits the machine control signal on the basis of the exercise points to the exercise machine, and changes the operation state of the exercise machine. Therefore, the exercise machine can be operated in an operation state corresponding to the exercise level of the user, whereby the exercise effect can be increased. Further, since the user can step up the operation state of the exercise machine by accumulating the exercise points, the user is continuously motivated to exercise, whereby the user's health can be promoted.

According to the invention as in claim 2, the exercise points for each user that have been determined by the server of the service provider are transmitted to an administrator of the community or user himself, whereby the user can be motivated to exercise.

According to the invention as in claim 3, the result obtained by totaling the exercise points of all of the residents living in the community are displayed on an advertisement for new residents to the community. Therefore, when the cumulative total number of exercise points can be increased by motivating the residents to exercise, the evaluation of activity state in the community is increased and the advertising effect of the advertisement can be increased.

According to the invention as in claim 4, the operation time interval of the user and the measurement data of health assessment indexes collected by the server of the service provider are provided to a physical therapist. As a result, the health state of the user is diagnosed by the physical therapist and the result is transmitted to the community administrator, the user, or the user's family. The advantage of such a feature is that the results of objective evaluation of the health state of the users can be obtained and measures aimed at maintaining and improving the health state can be easily taken.

In the insurance company providing medical insurance, when the health state of the insured person is good, the probability of the insured person being treated in a medical institution is decreased and the payment for medical care is expected to be reduced. According to the invention as in claim 5, when the diagnostic result obtained by the physical therapist is transmitted from the server of the service provider to the computer of the insurance company, a reduction ratio of the insurance premium is calculated on the basis of the diagnostic result. Thus, the residents of the community will try to exercise actively by using the exercise machine in order to maintain the health state and therefore they can be encouraged to use the exercise machine on a continuous basis.

### BRIEF DESCRIPTION OF THE DRAWINGS

FIG. 1 is a system configuration diagram illustrating a schematic configuration of the exercise machine system of the first embodiment of the present invention.
FIG. 2 is an external view of a passive exercise machine used in the exercise machine system of the first embodiment.
FIG. 3 is a block diagram of the passive exercise machine used in the exercise machine system of the first embodiment.
FIG. 4 is a block diagram of a center server used in the exercise machine system of the first embodiment.
FIG. 5 is an explanatory drawing illustrating the operation of the exercise machine system of the first embodiment.
FIG. 6 is a system configuration diagram illustrating a schematic configuration of the exercise machine system of the second embodiment.
FIG. 7 is a block diagram of the passive exercise machine used in the exercise machine system of the second embodiment.
FIG. 8 is a system configuration diagram illustrating a schematic configuration of the exercise machine system of the third embodiment.
FIG. 9 is a system configuration diagram illustrating a schematic configuration of the exercise machine system of the fourth embodiment.
FIG. 10 is a system configuration diagram illustrating a schematic configuration of the exercise machine system of the fifth embodiment.
FIG. 11 is a system configuration diagram illustrating another configuration of the exercise machine system of the fifth embodiment.

### BEST MODE FOR CARRYING OUT THE INVENTION

### (First Embodiment)

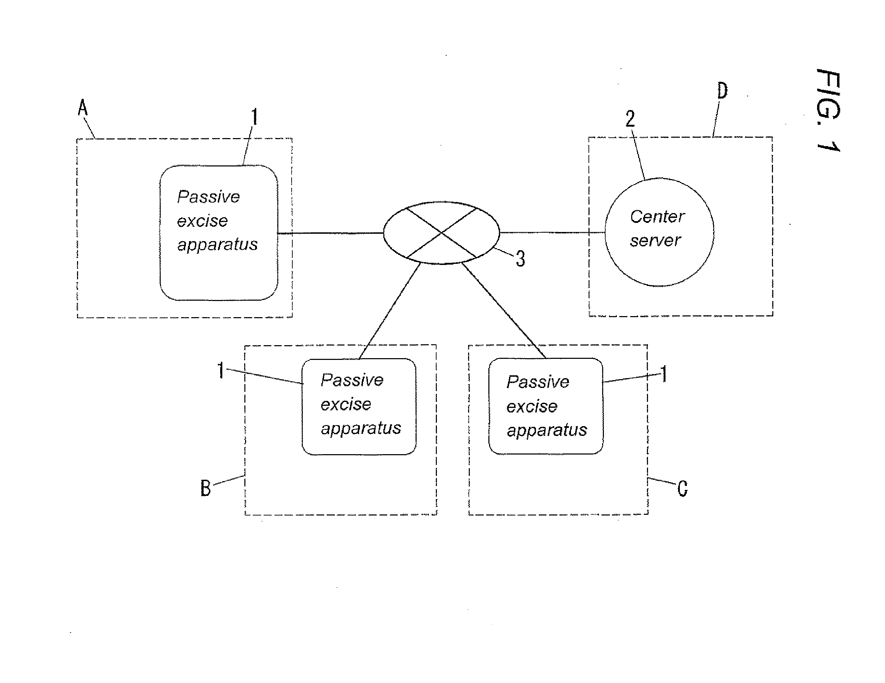

FIG. 1 shows a schematic system configuration of the exercise machine system of the first embodiment of the present invention. This exercise machine system is constituted by a plurality of passive exercise machines 1 and a center server 2. The passive exercise machines 1 are disposed in an individual home A, fitness center B, or a wellness center provided in a community C such as an AARC (Active Adult Retirement Communities) in which residents are provided with a living environment enabling the residents themselves to lead an active life. The center server 2 is operated by a service provider D that signed with an administrator of the community C a contract for services according to which the residents of the community C perform passive exercises using the passive exercise machines 1, whereby the health of the users (residents) of the passive exercise machines 1 is promoted. The passive exercise machines 1 use a middleware technology for internet connection of a machine incorporation type that is called EMIT® (Embedded Micro Internetworking Technology), and the operation of the passive exercise machines can be remotely monitored or controlled. Data communication between the passive exercise machine 1 used by the user who signed the service contact with the service provider D and the center server 2 is performed via a network 3 composed of a wide area network (WAN) such as Internet.

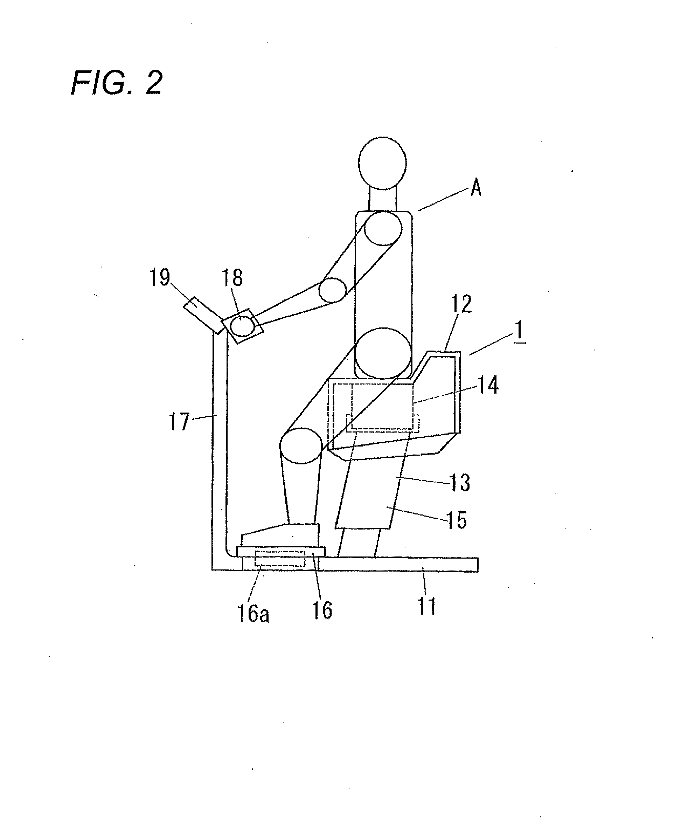

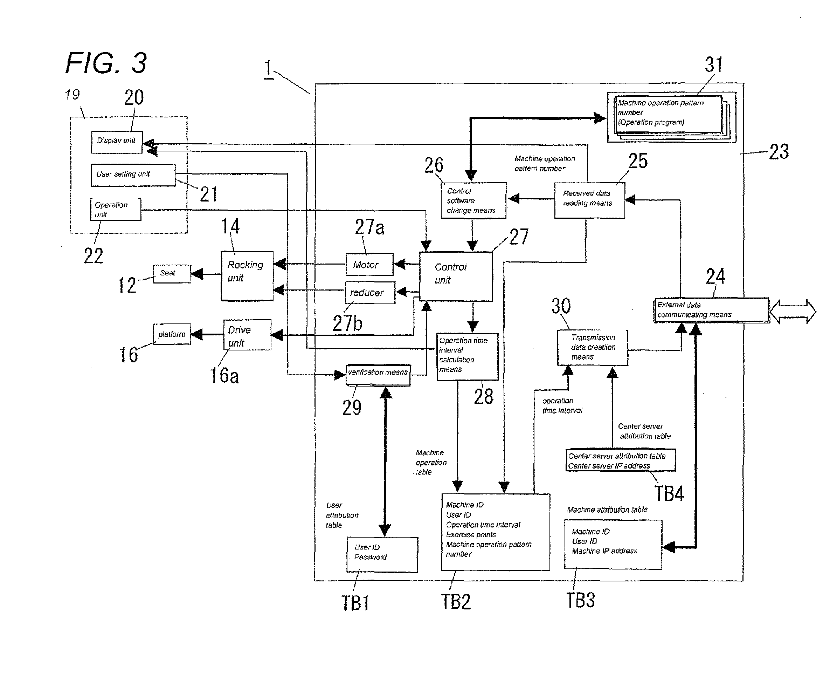

FIG. 2 shows a schematic external view of the passive exercise machine 1. The passive exercise machine 1 is provided with a support base 11 placed on the floor, and a rocking support unit 13 that swingably supports a seat 12 for a person A is disposed in the rear portion of the support base 11. The rocking support unit 13 is constituted by a rocking unit 14 and a support pillar 15. The rocking unit 14 allows the person to perform an exercise developing the muscle strength, for example, of abdominal muscles, back muscles, and femoral muscles, or an exercise improving the balance and posture of the trunk by rocking the seat 12 in the directions of three axes. The support pillar 15 has a height-adjusting mechanism and supports the rocking unit 14. The support base 11 is provided at its top surface in the front side with platforms 16 onto which the person A sitting on the seat 12 places the left and right feet. The left and right platforms 16 are individually driven to move up and down by a drive unit 16a, thereby enabling the exercise that strengthens muscles of legs, for example, knees and thighs. A support pillar 17 is provided vertically in the front end portion of the upper surface of the support base 11. Handles 18 grasped by the person A sitting in the seat 12 and a user interface device 19 are attached to the upper portion of the support pillar 17. The user interface device 19 is constituted by a display unit 20, a user setting unit 21, and an operation unit 22. The display unit 20 is composed of a liquid crystal display for providing information such as text or images. The user setting unit 21 specifies the user by referring to the biometrics information such as fingerprints. The operation unit 22 is composed of a plurality of operation switches (see FIG. 3).

FIG. 3 is a schematic block diagram of the passive exercise machine 1. This figure shows that the machine is mainly constituted by the rocking unit 14 that causes the seat 12 to rock, the user interface device 19, and a machine control unit 23 that performs the overall control.

The machine control unit 23 is provided with an external data communication means 24, a received data reading means 25, a control software change means 26, a control unit 27, an operation time interval measurement means 28, a verification means 29, a transmission data creation unit 30, a storage unit 31, a user attribution table TB1, a machine operation table TB2, a machine attribution table TB3, and a center server attribution table TB4. The external data communication means 24 is configured to perform data communication with the center server 2 via the network 3. The received data reading means 25 is configured to read the received data such as a machine operation pattern number or exercise points from the data received by the external data communication means 24. The control software change means 26 is configured to change the control software on the basis of the machine operation pattern number read by the received data reading means 25. The control unit 27 is configured to execute the control software changed by the control software change means 26 on the basis of an operation input from the operation unit 22, causing the rocking unit 14 to rock with a desired pattern (rocking angle, rocking speed, and rocking frequency) by controlling the revolution speed of a motor 27a and a gear ratio of a reducer 27b, as well as causing the platforms 16 to perform the desired action by controlling the operation of the drive unit 16a. The operation time interval measurement means 28 is configured to measure the operation time interval by counting the operation time of the rocking unit 14 and the drive unit 16a and writing the measured operation time interval in the machine operation table TB2. The verification means 29 is configured for personal verification on the basis of the biometrics information input from the user setting unit 21. The transmission data creation unit 30 is configured to create transmission data on the basis of a user ID (user identifier) read from the machine operation table TB2 when the user performs an exercise using the passive exercise machine 1, machine operation pattern number, and operation time interval and transmits the generated data from the external data communication means 24. The storage unit 31 is configured to store therein a plurality of kinds of operation programs (a machine operation pattern number used as a machine control signal is allocated to each operation program) that have been set under conditions with mutually different exercise conditions such as load, speed, and rocking period. The user attribution table TB1 (identifier storage means) is configured to store therein attribution information of each user (user ID or password). The machine operation table TB2 (measured data storage means) is configured to store therein operation information (operation time interval, exercise points obtained by the user, machine operation pattern of the operation program that has been used, and the like) of the passive exercise machine 1 in association with a machine ID (machine identifier) of the respective machine and a user ID of the user. The machine attribution table TB3 is configured to store therein attribution information (a machine ID of the respective machine, a user ID of the user, an IP address allocated to the machine (referred to hereinbelow as machine IP address)) of the passive exercise machine 1. The center server attribution table TB4 is configured to store therein attribution information (IP address of the center server 2 (referred to hereinbelow as server IP address) and the like) of the center server 2 that is operated by a service provider D. The above means 25 to 30 of the machine control unit 23 are herein realized by computational functions of a computer when control programs that have been incorporated in advance in the passive exercise machine 1 are executed by the machine control device 23. Exercise load application means is constituted by the rocking unit 14 that causes the seat 12 to rock and the drive unit 16a that drives the platforms 16. Operation control means is constituted by the control software change means 26 and the control unit 27. The left and right platforms 16 are driven by the drive unit 16a. In another possible configuration, the platforms 16 are supported by springs instead of being driven by the drive unit 16a, and the exercise is performed by stepping on the platforms 16 with the left and right foot under the effect body weight transfer corresponding to the rocking movement of the seat 12.

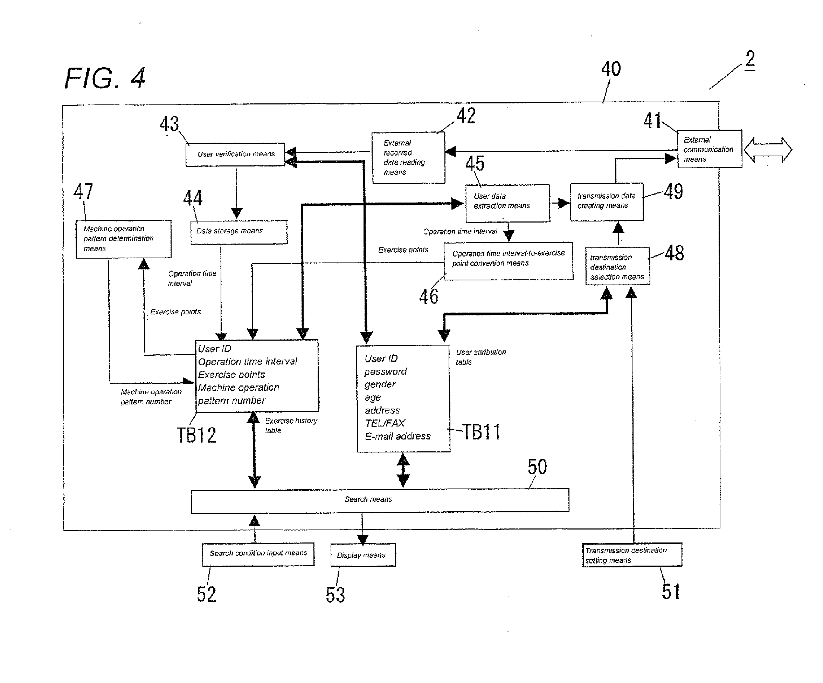

FIG. 4 shows a schematic block diagram of the center server 2 that is mainly constituted by a server control unit 40, transmission destination setting means 51, search condition input means 52, and display means 53. The server control unit 40 is provided with a user attribution table TB11, an exercise history table TB12, external communication means 41, external received data reading means 42, a user verification means 43, a data storage means 44, a user data extraction means 45, operation time interval-to-exercise point conversion means 46, a machine operation pattern determination means 47, a transmission destination selection means 48, and a search means 50. The user attribution table TB11 has a predetermined attribution information (user ID, password, gender, age, location, phone number, fax number, Email address, and the like) for each individual user (residents of the community C). The exercise history table TB12 is configured to store therein history information (operation time interval, exercise points, machine operation pattern number, and the like) of the exercise performed by each user in association with the user ID. The external communication means 41 is configured to perform data communication via the network 3. The external received data reading means 42 is configured to read the received data such as the user ID and operation time interval from the data received by the external communication means 41. The user verification means 43 is configure to verify whether the user is a user that has been registered in advance, on the basis of the user ID and password read by the external received data reading means 42 by using the user attribution table TB11. The data storage means 44 is configured to store therein the user ID and operation time integral that have been read by the external received data reading means 42 in the exercise history table TB12 if the verification is successful. The user data extraction means 45 is configure to extract data (operation time interval and the like) of the user that has been verified from the exercise history table TB12. The operation time interval-to-exercise point conversion means 46 is configured to convert the operation time interval extracted by the user data extraction means 45 from the exercise history table TB12 into the exercise points and registering the exercise points in the exercise history table TB12. The machine operation pattern determination means 47 is configured to determine a machine operation pattern number during next exercise cycle on the basis of the present machine operation pattern number and a cumulative total number of exercise points registered in the exercise history table TB12 and updating the machine operation pattern number registered in the exercise history table TB12. The transmission destination selection means 48 is configured to select an Email address of transmission destination by referring to the user attribution table TB11 if an operator of the center server 2 sets the transmission destination by using the transmission destination setting means 51. The search means 50 is configured to extract information matching the search conditions from the user attribution table TB11 and the exercise history table TB12 and displaying the extracted information on the display means 53 if the operator inputs the search conditions by using the search condition input means 52. The above means 42 to 49 are realized by computational functions of a computer when control programs that have been incorporated in advance in the center server 2 are executed. Server storage means is constituted by the user attribution table TB11 and the exercise history table TB12.

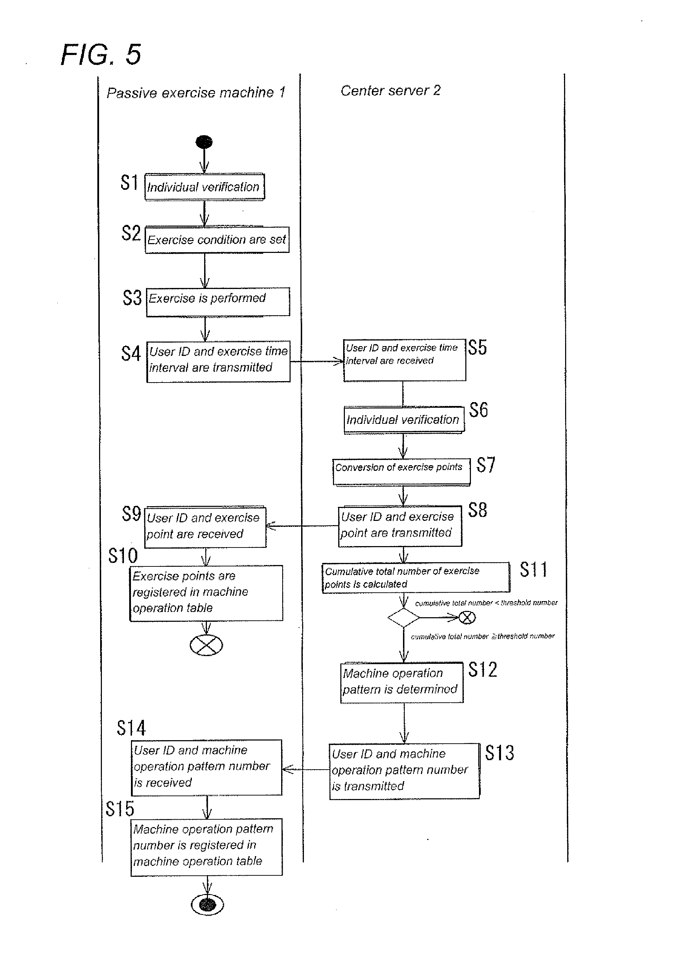

With reference to the activity diagram shown in FIG. 5, explanations are made below as to the operation of exercise machine system of the present embodiment when an exercise load of the passive exercise machine 1 is changed in the center server 2 to match the exercise level of the user of the passive exercise machine 1. An operation program that has been set, for example, to three levels of exercise intensity (low, moderate, high) by changing the rocking angle, rocking speed, and rocking period of the seat 12 or the rocking angle, amplitude, or period of the platforms 16 is stored in the storage unit 31 of the passive exercise machine 1. The exercise level of the user is determined in the center server 2, and the operation program of the passive exercise machine 1 is changed to match the exercise level of individual users.

When the user of the passive exercise machine 1 mounts the seat 12 and inputs his own biometrics information into the user setting unit 21 provided in the user interface device 19, the verification means 29 of the machine control unit 23 performs individual verification on the basis of the biometrics information input from the user setting unit 21 (step S1). When the verification is successful, the user ID and password of the user are read from the user attribution table TB1. The user then operates the operation unit 22 and sets the exercise conditions (step S2). When a start operation is performed, the control unit 27 controls the rocking unit 14 and the drive unit 16a for performing a passive exercise. In this case, the operation time interval (same as the exercise time interval) is measured by the operation time interval measurement means 28 and the measured operation time interval is stored in the machine operation table TB2 (step S3). When the user then performs an end operation by using the operation unit 22, the control unit 27 stops the rocking unit 14 and the drive unit 16a and ends the passive exercise. When the passive exercise performed with the passive exercise machine 1 ends, the transmission data creation means 30 of the machine control unit 23 reads the operation time interval, user ID, and password from the machine operation table TB2 as well as the server IP address from the center server attribution table TB4. Then, the transmission data creation means 30 of the machine control unit 23 creates the transmission data (include the user ID, password, and operation time interval), and transmits the transmission data to the center server 2 via the external data communication means 24 (step S4).

In this case, in the center server 2, the external communication means 41 receives the data transmitted from the passive exercise machine 1 (step S5), the external received data reading means 42 reads the user ID, password, and operation time interval from the received data, and the user verification means 43 performs individual verification by refereeing to the data stored in the user attribution table TB11 (step S6). When the individual verification is successful, the data storage means 44 stores the operation time interval in the exercise history table TB12 in association with the user ID. At the same time, the user data extraction means 45 extracts the operation time from the exercise history table TB12. Then, the number of exercise points is calculated in the operation time interval-to-exercise point conversion means 46, for example, by multiplying the extracted operation time interval by a predetermined coefficient (step S7). Next, the obtained exercise points are associated with the user ID and stored in the exercise history table TB12. Once the exercise point conversion processing has been completed, the user data extraction means 45 extracts the user ID of the user and the exercise points from the exercise history table TB11. Then, transmission data are created by the transmission data creation means 49. Subsequently, the created transmission data are transmitted to the corresponding passive exercise machine 1 via the external communication means 41 (step S8).

In the passive exercise machine 1, when the external data communication means 24 receives the data transmitted form the center server 2 (step S9), the received data reading means 25 reads the exercise points contained in the received data, and displays the exercise points at the display unit 20. The exercise points are associated with the user ID to be stored in the machine operation table TB2 (step S10).

Further, in the center server 2, after the exercise points have been received, the machine operation pattern determination means 47 reads history information of the exercise points from the exercise history table TB12, determines a cumulative total number of the exercise points (step S11), and determines whether the cumulative total number is equal to or higher than a predetermined threshold. When the cumulative total number of exercise points is determined to be less than the threshold, the machine operation pattern determination means 47 terminates the processing without changing the machine operation pattern number. When the cumulative total number of exercise points is equal to or higher than the threshold, the machine operation pattern determination means 47 changes the machine operation pattern number to one corresponding to the cumulative total number of the exercise points, and registers the changed machine operation pattern number in the exercise history table TB12 (step S12). Further, in the center server 2, when the machine operation pattern number is changed, the user data extraction means 45 extracts the changed machine operation pattern number and the user ID from the exercise history table TB12. Then, the transmission data creation means 49 creates transmission data on the basis of the extracted machine operation pattern number and user ID, and the created data are transmitted to the corresponding passive exercise machine 1 via the external communication means 41 (step S13).

In the passive exercise machine 1, after the external data communication means 24 receives the data transmitted from the center server 2 (step S14), the received data reading means 25 reads the user ID and machine operation pattern number contained in the received data. Then, the machine operation pattern number is associated with the user ID to be stored in the machine operation table TB2 (step S15). Therefore, by performing the passive exercise corresponding to the changed machine operation pattern number in the next exercise cycle, the passive exercise makes it possible to provide the exercise load matching the exercise level of the user.

In this case, the center server 2 converts the operation time interval of each user transmitted form the passive exercise machine 1, into exercise points. Then, the center server 2 changes a machine operation pattern on the basis of the cumulative total number of the exercise point. The cumulative total number of the exercise points indicates the amount of exercise for the passive exercise that the user has heretofore performed by using the passive exercise machine 1. When the cumulative total number of exercise points becomes equal to or higher than the predetermined threshold, the exercise level of the user can be determined to exceed a certain reference value. Therefore, the effect of passive exercise can be increased by changing the machine operation pattern to one with a higher exercise intensity. Further, since the machine operation in excess of the exercise level of the user is not used, no unnecessary load is applied to the user's body. When the user continues to use the passive exercise machine 1, the exercise level of the user will increase gradually. In the present system, the center server 2 determines the exercise points of each individual user using the passive exercise machine 1. When the number of exercise points exceeds the predetermined threshold, the exercise load in using the passive exercise machine 1 is changed to one with a higher exercise intensity and the cumulative total number of exercise points is presented to the user. Therefore, the user will try to accumulate a larger number of exercise points in order to step up the machine operation pattern. As a result, the user will act to perform actively the passive exercise by using the passive exercise machine 1 and therefore the user will be motivated to move and the user's health can be promoted.

In the present embodiment, the passive exercise machine 1 with the structure shown in FIG. 2 is explained by way of example, but the passive exercise machine 1 is not limited to the present embodiment. Further, the passive exercise machine 1 for allowing the user to perform a passive exercise is explained by way of example, but an exercise machine for allowing the user to perform an automatic exercise may be also used.

### (Second Embodiment)

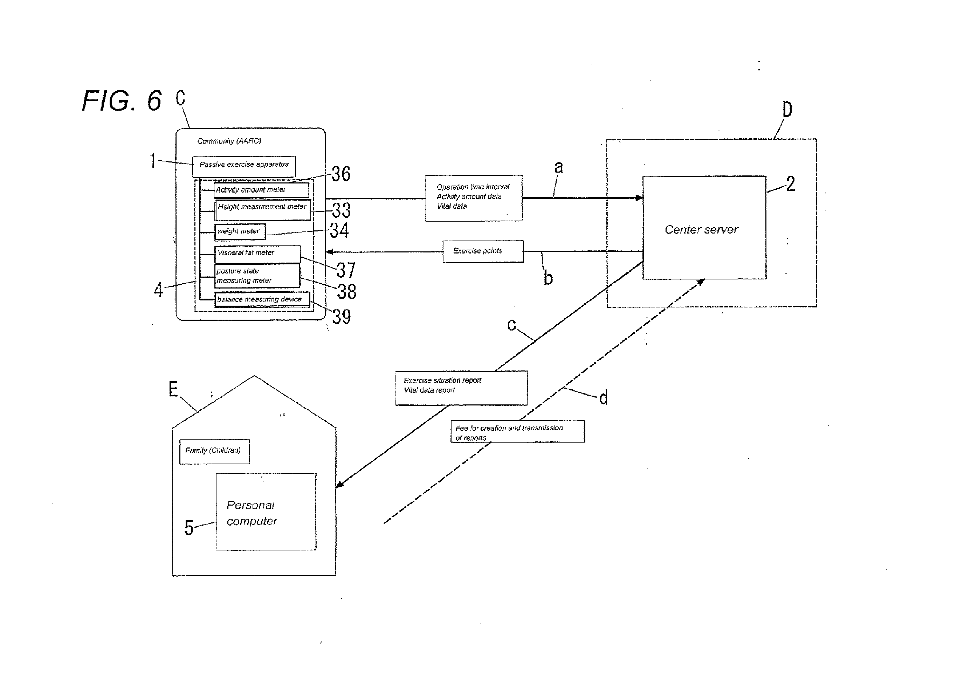

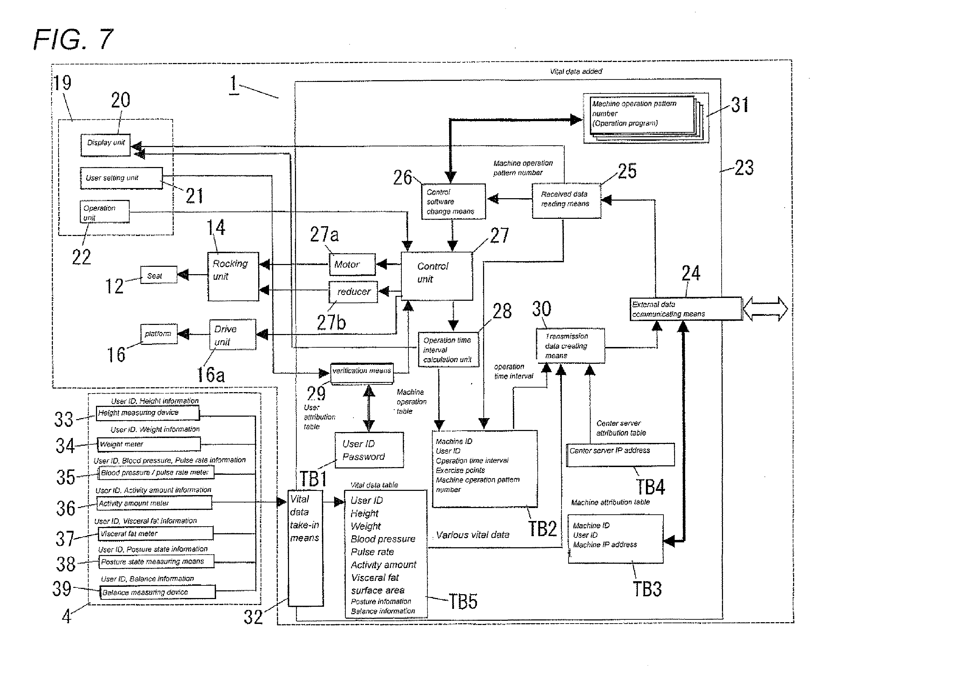

The second embodiment of the present invention will be described with reference to FIG. 6 and FIG. 7. FIG. 6 is a schematic system configuration diagram of the exercise machine system. The present system is constituted by a passive exercise machine 1, a center server 2, and a personal computer (abbreviated hereinbelow as PC) 5. The passive exercise machine 1 is disposed in a wellness center in a community C, for example, such as AARC. The center server 2 is operated by a service provider D that provides services for health management and health promotion by using the passive exercise machines 1 to the users living in the community C. The personal computer (abbreviated hereinbelow as PC) 5 has a network communication function and is disposed in a home E of the user living in the community C. The EMIT technology is used in the passive exercise machine 1, and the machine operation can be remotely monitored and controlled. Data communication is performed between the passive exercise machine 1 that is used by the user who signed a contract for using the aforementioned services with the service provider D and the center server 2 via a network 3 constituted by a wide area network (WAN) such as Internet. The configurations of the passive exercise machine 1 and the center server 2 are substantially identical to those explained in the first embodiment. Therefore, common constituent elements will be assigned with same reference numerals and explanation thereof will be omitted.

A health state measurement unit 4 that measures health assessment indexes (vital data, physical constitution, and data on exercise capability) evaluating the health state of the user is disposed in the wellness center of the community C close to the location of the passive exercise machine 1, as shown in FIG. 6 and FIG. 7. The health state measuring unit 4 in the present embodiment comprises a height measuring device 33 that measures the height of the user, a weight meter 34 that measures the weight, a blood pressure/pulse rate meter 35 that measures the blood pressure and pulse rate, an activity amount meter 36 that measures the amount of activity, a visceral fat meter 37 that measures visceral fat, a posture state measuring device 38 that measures a standing posture, and a balance measuring device 39 that measures balance functions of the user. The machine control unit 23 of the passive exercise machine 1 is provided with a vital data take-in means 32 and a vital data table TB5. The vital data take-in means 32 takes in the measurement results from the height measuring device 33, weight meter 34, blood pressure/pulse rate meter 35, activity amount meter 36, visceral fat meter 37, posture state measuring device 38, and balance measuring device 39. The vital data table TB5 is provided to store therein, in association with the user ID, various measurement data that have been taken by the vital data take-in means 32. The posture state measuring device 38 has a detection unit in which a plurality of vertically arranged contacts provided so that they can be moved back and forth in the horizontal direction. When the tips of the contacts are pressed from a side against the back of the standing person, the contacts move back and forth according to the degree of curvature of the back. Therefore, peaks and valleys present on the back of the person are detected from the positions of the contacts, thereby detecting the standing posture of the person. The balance measuring device 39, for example, has an image pick-up device that picks up the images of the walking or moving person and the person's image picked up by the image pick-up device can be subjected to image processing to measure the balance during walking or moving.

A procedure for performing health administration of the user with the present system will be explained below. The user living in the community C measures the health assessment indexes by using the health state measurement unit 4 in the wellness center. When the user then performs a passive exercise by using the passive exercise machine 1, once the exercise is completed, the transmission data creation means 30 of the passive exercise machine 1 reads the operation time interval from the machine operation table TB2, reads measurement data for each evaluation item from the vital data table TB5, then reads the server IP address from the center server attribution table TB4, and generates transmission data (including the user ID, password, various measurement data, and operation time interval), and the generated data are transmitted to the center server 2 via the external data communication means 24 ("a" in FIG. 6).

After external communication means 41 of the center server 2 receives the data transmitted from the passive exercise machine 1, the external received data reading means 42 reads the user ID, password, various measurement data, and operation time interval from the received data. Then, the user verification means 43 performs individual verification by referring to the data stored in the user attribution table TB11. When the individual verification is successful, the data storage means 44 stores therein the received operation time interval and various measurement data in association with the user ID in the exercise history table TB12. At the same time, the user data extraction means 45 extracts the operation time interval from the exercise history table TB12. Then, the operation time interval-to-exercise point conversion means 46 converts the extracted operation time interval into exercise points. The exercise points are stored in association with the user ID in the exercise history table TB12. When the conversion of the exercise points is completed, the user data extraction means 45 extracts the user ID and exercise points of the user from the exercise history table TB11, transmission data are created by the transmission data creation means 49. Next, the generated transmission data are transmitted to the corresponding passive exercise machine 1 via the external communication means 41 ("b" in FIG. 6).

When the external data communication means 24 in the passive exercise machine 1 receives data transmitted from the center server 2, the received data reading means 25 reads the exercise points contained in the received data and the exercise points are displayed on the display unit 20 and also stored in association with the user ID in the machine operation table TB2.

Further, in the center server 2, after the transmission of the exercise points, the machine operation pattern determination means 47 reads history information on exercise points from the exercise history table TB 11, determines the cumulative total number of exercise points, and determines whether the cumulative total number is equal to or higher than the predetermined threshold. When the cumulative total number of exercise points is less than the threshold, the machine operation pattern determination means 47 terminates the processing without changing the machine operation pattern number. When the cumulative total number of exercise points is equal to or higher than the threshold, the machine operation pattern number is changed to one corresponding to the cumulative total number of exercise points. The changed machine operation pattern number is registered in the exercise history table TB12. After the center server 2 changes the machine operation pattern number, the user data extraction means 45 extracts the changed machine operation pattern number and user ID from the exercise history table TB12. The transmission data generation means 49 generates transmission data on the basis of the extracted machine operation pattern number and user ID. The transmission data are transmitted to the corresponding passive exercise machine 1 via the external communication means 41.

After the external data communication means 24 in the passive exercise machine 1 receives the data transmitted from the center server 2, the received data reading means 25 reads the user ID and machine operation pattern number contained in the received data. Then, the machine operation pattern number is stored in association with the user ID in the machine operation table TB2. Therefore, an exercise load matching the exercise level of the user can be provided by performing a passive exercise corresponding to the changed machine operation pattern number in the next exercise cycle.

Further, the center server 2 generates an exercise situation report indicating an exercise situation in a predetermined reporting period (for example, one month) and a vital data report indicating measurement data for each evaluation item in the reporting period each time the reporting period ends on the basis of the history of operation time interval and measurement data on various health assessment indexes stored in the exercise history table TB12. At the same time, the transmission destination selection means 48 reads an Email address of the transmission destination from the user attribution table TB11. The transmission data generation means 49 generates an Email having the two aforementioned report files attached thereto, transmitting the generated Email via the external communication means 41, and thereby periodically transmitting the exercise situation report and vital data report to the PC 5 disposed in the home E ("c" in FIG. 6). The family living in the home E signs a delivery contract relating to the exercise situation report and vital data report with the service provider D and pays a predetermined fee (monthly payment or single payment) to the service provider D ("d" in FIG. 6) for the transmission of the reports.

Each time the user living in the community C performs a passive exercise by using the passive exercise machine 1, the center server 2 accumulates the performed exercise points or the history of measurement data for each evaluation item which is measured by the health state measurement unit 4 when the exercise is started. Then, the center server 2 periodically creates the exercise situation report and vitality report for each individual on the basis of the accumulated data and delivered to the PC 5 of the family. Therefore, the family of the resident of the community C can get information about the exercise situation or health state of the resident. The created exercise situation report or vitality report may be also delivered by electronic mail to the PC located in the community C or a cellular phone of the user. It enables the user himself to verify the exercise situation or health state. As a result, the user is motivated to exercise and encouraged to use the passive exercise machine 1 on a continuous basis.

### (Third Embodiment)

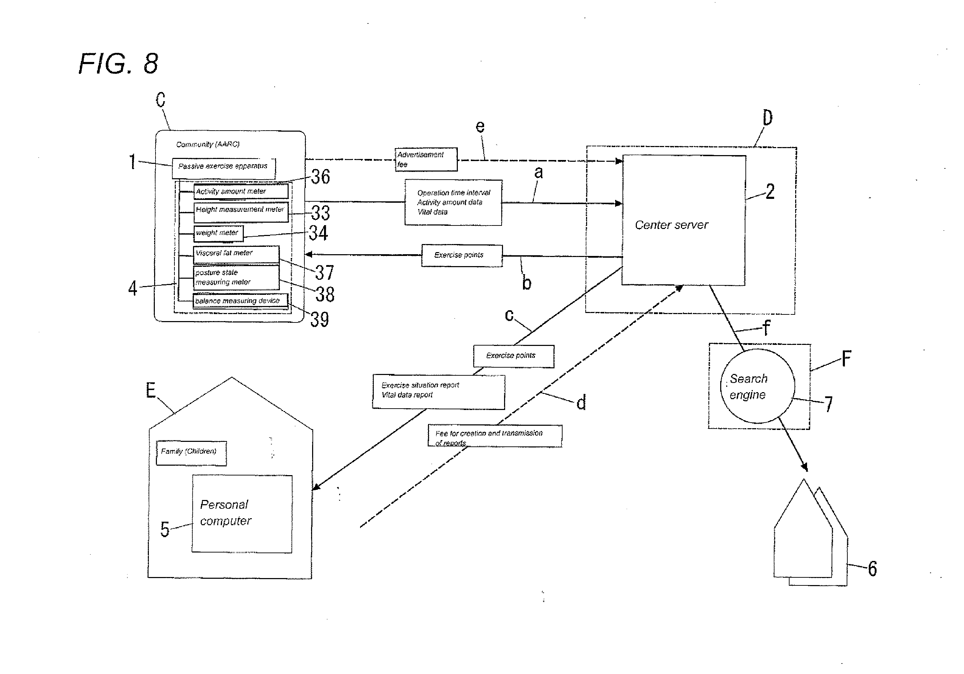

The third embodiment of the present invention will be explained below with reference to the drawings. FIG. 8 is a schematic system configuration diagram of the exercise machine system. The present system is constituted by a passive exercise machine 1, a center server 2, a PC 5, and a server 7. The passive exercise machine 1 is disposed in a wellness center in a community C, for example, such as AARC. The center server 2 is operated by a service provider D that provides services for health management and health promotion by using the passive exercise machine 1 to the user living in the community C. The PC 5 has a network communication function and disposed in a home E of the user living in the community C. the server 7 is operated by an operator F who establishes a search site on the Internet. The EMIT technology is used in the passive exercise machine 1 and the machine operation can be remotely monitored and controlled. Data communication is performed between the passive exercise machine 1 that is used by the user who signed a contract for using the aforementioned services with the service provider D and the center server 2 via a network 3 constituted by a wide area network (WAN) such as Internet. The configurations of the passive exercise machine 1 and the center server 2 are substantially identical to those explained in the second embodiment. Therefore, common constituent elements will be assigned with same reference numerals and explanation thereof will be omitted. In the present embodiment, the processing performed between the passive exercise machine 1 and a health state measurement unit 4 disposed in the wellness center of the community C, the center server 2, and the PC 5 disposed in the home E is also similar to that explained in the second embodiment. Therefore, the explanation thereof will be omitted.

In the present system, the service provider D receives a request for an advertisement for new residents to the community C from the community that has signed a contract for use of the aforementioned services and receives the payment of the advertisement fee ("e" in FIG. 8). The service provider D then request an Internet advertisement from an operator F (advertiser) of a search site such as Google® or Yahoo®. When a general user accesses a web site 7 by using a PC 6 having a browser installed therein and performs a search by relevant keywords, the Internet advertisement introducing the community C will be displayed on the display screen of search results. Each time the Internet advertisement is displayed, the operator F (advertiser) of the search site demands the payment of an advertisement fee from the service provider D.

In this configuration, since the cumulative total number of exercise points has been accumulated for all of the users living in the community C in the center server 2, a server control unit 40 updates appropriately the advertisement contents so as to display the sum of the cumulative total number of exercise points for all of the users in the Internet advertisement and reports the updated contents to the advertiser F ("f" in FIG. 8). Since a large number of exercise points indicates an active lifestyle of the residents of the community C, the effect of soliciting to join the community can be increased.

The advertisement fee demanded from the community C by the service provider D is determined by the server control unit 40. For example, the server control unit 40 may determine a discount correspondingly to the cumulative total number of exercise points for requesting the community C to pay an amount obtained by subtracting the discount from the basic fee as the advertisement contract fee. In this case, by encouraging the use of the passive exercise machine 1 and motivating the use on a continuous basis, it is possible to retain the customers and ensure continuous sales in the form of maintenance fees and payment for updating the equipment.

### (Fourth Embodiment)

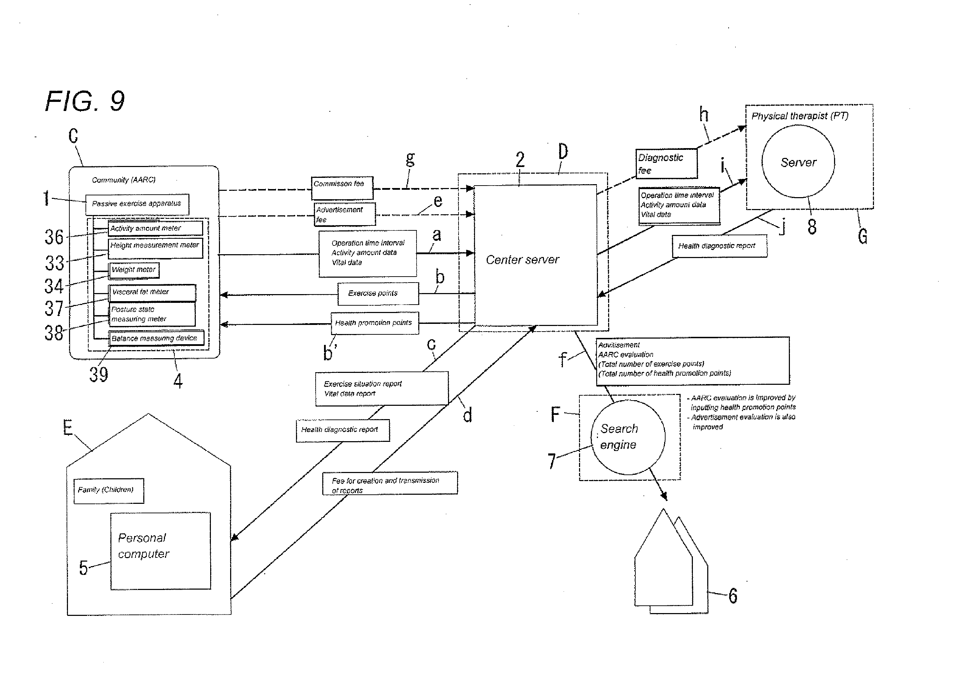

The fourth embodiment of the present invention will be explained below with reference to the drawings. FIG. 9 is a schematic system configuration diagram of the exercise machine system. The present system is constituted by a passive exercise machine 1, a center server 2, a PC 5, and a server 8. The passive exercise machine 1 is disposed in a wellness center in a community C, for example, such as AARC. The center server 2 is operated by a service provider D that provides services for health management and health promotion by using the passive exercise machine 1 to the user living in the community C. the PC 5 has a network communication function and disposed in a home E of the user living in the community C. The server 8 is constituted by a computer having a Web server function and provided at a workplace of a physical therapist (PT) G. The EMIT technology is used in the passive exercise machine 1 and the machine operation can be remotely monitored and controlled. Data communication is performed between the passive exercise machine 1 that is used by the user who signed a contract for using the aforementioned services with the service provider D and the center server 2 via a network 3 constituted by a wide area network (WAN) such as Internet. The configurations of the passive exercise machine 1 and the center server 2 are substantially identical to those explained in the second embodiment. Therefore, common constituent elements will be assigned with same reference numerals and explanation thereof will be omitted. The processing performed between the passive exercise machine 1 and a health state measurement unit 4 disposed in the wellness center of the community C, the center server 2, and the PC 5 disposed in the home E is similar to that explained in the second embodiment, and the processing of providing an Internet advertisement from the center server 2 to a search site 7 operated by an operator F is similar to that explained in the third embodiment. Therefore, the explanation of such processing will be omitted.

In the present system, the user data extraction means 45 of the center server 2 periodically (for example, once a week) extracts data (namely, the operation time interval in which the passive exercise machine 1 has been used and the measurement data of health assessment indexes measured by a health state measurement unit 4) relating to each user that have been accumulated in an exercise history table TB12. Then, transmission data creation means 49 creates transmission data on the basis of the extracted data. Subsequently, the transmission data are transmitted to an IP address of a server 8 selected by transmission destination selection means 48, via external communication means 41 ("i" in FIG. 9).

The server 8 of the physical therapist G receives the data transmitted from the center server 2 and displays the received data on a display screen. The server 8 of the physical therapist G determines the health state of the user on the basis of the data displayed on the screen and results obtained in interviewing the user, then creating a health diagnostic report, and subsequently determining health promotion points that quantify the health level of the user. The physical therapist G then operates the server 8, designates the IP address of the return destination, and returns the data on health diagnostic report and health promotion points from the server 8 to the center server 2 ("j" in FIG. 9).

After receiving the data on health diagnostic report and health promotion points transmitted from the server 8 at external communication means 41, the server control unit 40 of the center server 2 accumulates the received data on health diagnostic report and health promotion points in the exercise history table TB12. Then, the server control unit 40 transmits transmission data including the user ID and the health promotion points from the external communication means 41 to the passive exercise machine 1 ("b'" in FIG. 9). Subsequently, the server control unit 40 associates the transmission data with the user ID to be stored in the machine operation table TB2 of the passive exercise machine 1. Since the health promotion points of all of the users have been accumulated in the exercise history table TB12 of the center server 2, the server control unit 40 updates appropriately the advertisement contents so as to display the sum of the health promotion points in addition to the sum of the exercise points for all of the users in the Internet advertisement and reports the updated contents to the advertiser F ("f" in FIG. 9). Since a large number of health promotion points indicates that the community C features a living environment enabling the residents to lead an active life, the effect of the advertisement for new residents to the community can be increased.

The server control unit 40 of the center server 2 transmits the data on health diagnostic report received from the physical therapist G, to the IP address of a Personal computer C 5 disposed in a home E. The server control unit 40 of the center server 2 transmits the health diagnostic report created by the physical therapist as well as the exercise situation report and vital data report created by the center server 2 to the family. Therefore, the family of the resident can determine, without leaving the home, the exercise situation or health state of the resident in a remote location. Further, since the health diagnostic report has been created by the physical therapist, credibility of the data transmitted from the center server 2 can be increased.

In this case, the community C pays a commission fee for hiring the physical therapist G as a specialist and a diagnostic fee to the service provider D operating the center server 2 ("g" in FIG. 9). In addition, the service provider D pays the diagnostic fee corresponding to the amount submitted with the health diagnostic report to the physical therapist G ("h" in FIG. 9).

### (Fifth Embodiment)

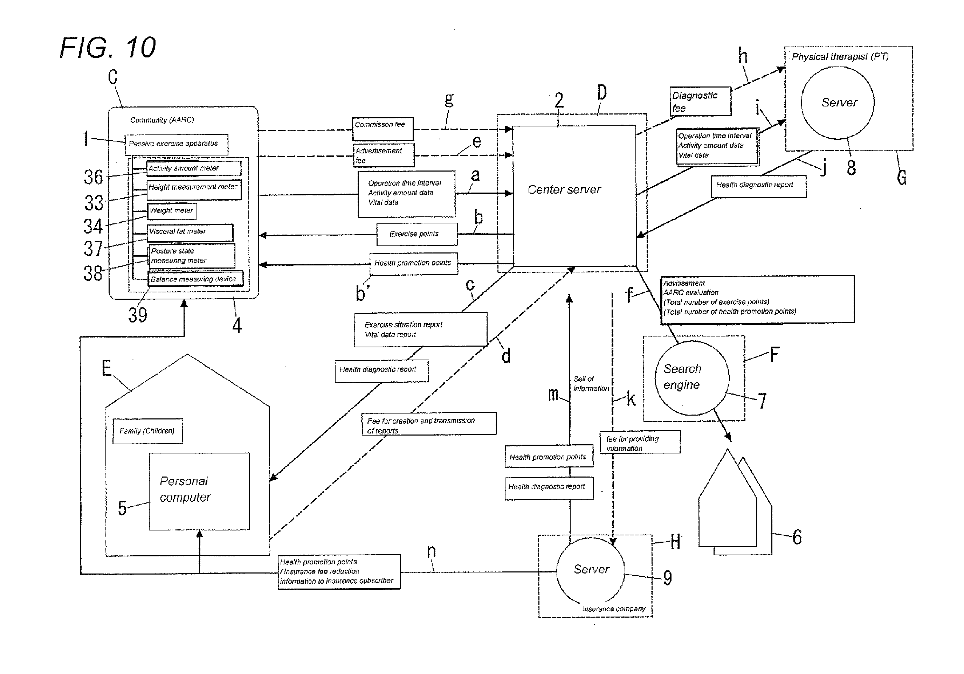

The fifth embodiment of the present invention will be explained below with reference to the drawings. FIG. 10 is a schematic system configuration diagram of the exercise machine system. The present system is constituted by a passive exercise machine 1, a center server 2, a PC 5, a server 8, and a server 9. The passive exercise machine 1 is disposed in a wellness center in a community C, such as AARC. The center server 2 is operated by a service provider D that provides services for health management and health promotion by using the passive exercise machine 1 to the user living in the community C. The PC 5 has a network communication function and disposed in a home E of the user living in the community C. The server 8 is constituted by a computer having a Web server function and provided at a workplace of a physical therapist (PT) G. The server 9 is provided in an insurance company H that has signed a medical insurance contact with the user living in the community C. The EMIT technology is used in the passive exercise machine 1 and the machine operation can be remotely monitored and controlled. Data communication is performed between the passive exercise machine 1 that is used by the user who signed a contract for using the aforementioned services with the service provider D and the center server 2 via a network 3 constituted by a wide area network (WAN) such as Internet. The configurations of the passive exercise machine 1 and the center server 2 are substantially identical to those explained in the second embodiment. Therefore, common constituent elements will be assigned with same reference numerals and explanation thereof will be omitted. The processing performed between the passive exercise machine 1 and a health state measurement unit 4 disposed in the wellness center of the community C, the center server 2, the PC 5 disposed in the home, and the server 8 of the physical therapist G is similar to that explained in the second to fourth embodiments, and the processing of providing an Internet advertisement from the center server 2 to a search site 7 operated by an advertiser F is similar to that explained in the third embodiment. Therefore, the explanation of such processing will be omitted.

The insurance company H enters into a medical insurance contract with the user living in the community C. When the insured person (resident of the community C) has a poor health state, payment to medical institutions and the like will increase. When the insured person is healthy, the payment for insurance coverage to the medical institutions or the like will be decreased. Therefore, the insurance company varies the insurance premium correspondingly to the health state of the insured person. When the insured person is healthy, the insurance premium is reduced, thereby making the insured person more aware about the health. In this case, although the insurance revenue is somewhat reduced, profits can be increased by reducing the amount of payment for insurance coverage. Accordingly, in the present system, the service provider D operating the center server 2 signs a contract with the insurance company H for providing information (for example, health diagnostic report and health promotion point) relating to the health state of the insured person. When the payment for providing information is received from the insurance company H ("j" in FIG. 10), the service provider transmits information relating to the user (insured person) who is the subject of contract to the server 9 of the insurance company H (FIG. 10).

The server 9 of the insurance company H is provided with a communication means (not shown) for receiving diagnostic results for each user transmitted from the center server 2 and an insurance premium calculation means (not shown) for calculating a deduction ratio of the insurance premium on the basis of the diagnostic results of each user. When the communication means receives the health promotion points of the insured person from the center server 2 and the health diagnostic report created by the physical therapist G for each user (each insured person), the premium fee calculation means determines insurance points that will be used in calculating the reduced insurance fee on the basis of the received health promotion point. Then, the premium fee calculation means calculates the reduced insurance fee from the insurance points. Thus, the insurance fee calculation means calculates a reduction ratio of the premium fee from the insurance point, and determines the reduced insurance fee by multiplying the insurance fee before the reduction by the fee reduction ratio. Since the insurance points are calculated on the basis of the health promotion points in the server 9 of the insurance company H, it is possible to transmit only the health promotion point from the center server 2 and omit the transmission of the health diagnostic report. However, since the insurance points are data used for calculating the reduced insurance fee, it is preferred that the health diagnostic report created by the physical therapist G be provided as support for calculation to the insurance company H.

When the insurance company H determines the reduced insurance premium for each user, the calculation result of the reduced insurance premium is delivered together with the health promotion points by an electronic mail to the PC in the community C or to the PC 5 in the home E. Therefore, each user or family thereof can be notified of both the health promotion points and the reduced insurance premium. Therefore, the user or family thereof can be notified of the reduction in the insurance premium resulting from the increase in the number of health promotion points caused by the passive exercise using the passive exercise machine 1. Therefore, the residents of the community C will actively perform passive exercises using the passive exercise machine 1 so as to reduce further the insurance premium. As a result, the number of exercise points and also the number of health promotion points will be further increased. Further, when the health state of the residents of the community C is improved, the residents will have higher resistance to illnesses, the number of admissions to medical institutions can be expected to decrease and a risk of serious illness is reduced. Therefore, although the medical insurance premium is reduced, long-term profits for the insurance company H can be increased by reducing the amount of payment for medical insurance coverage.

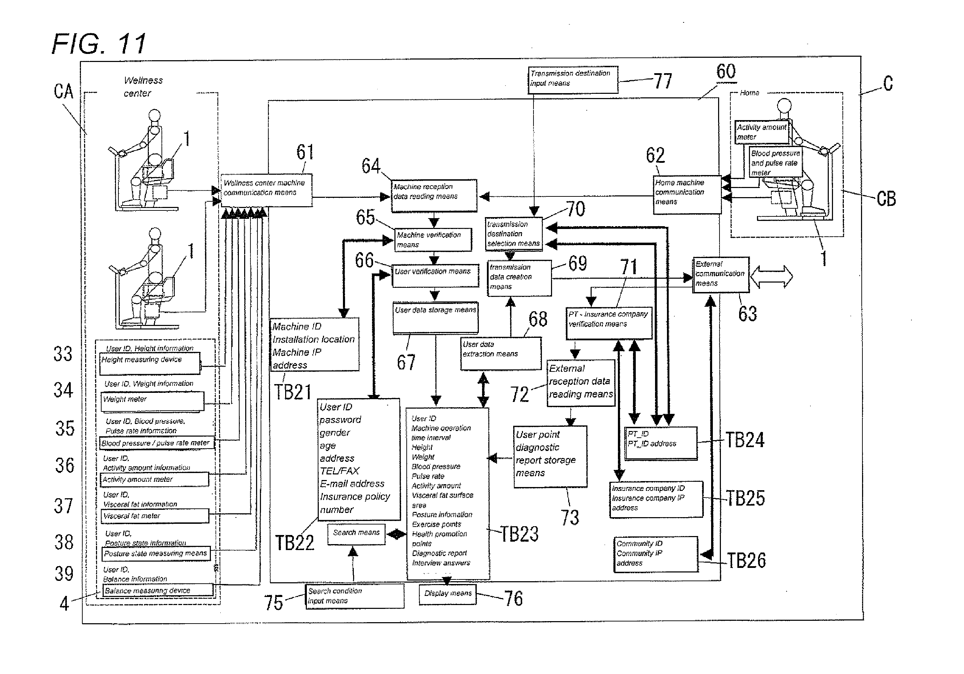

In the above-described embodiments, the passive exercise machine 1 is directly connected to the Internet by using a device-incorporating network technology (EMIT technology) in the passive exercise machine 1 disposed in the community C. In another possible configuration, a server 60 is disposed in the community C, as shown in FIG. 11, the connection by LAN is performed between the passive exercise machine 1, the health state measurement unit 4, and the server 60 disposed in a wellness center CA and between the passive exercise machine 1 and the server 60 disposed in a residence CB of each resident, and the server 60 transmits the operation time interval collected from the passive exercise machine 1 and the measurement data collected from the heath state measurement unit 4 to the center server 2.

In this case, individual device IDs are allocated respectively to the passive exercise machine 1 disposed in the wellness center CA or the residence CB, and health state measurement unit 4 (height measuring device 33, weight meter 34, blood pressure/pulse rate meter 35, activity amount meter 36, visceral fat meter 37, posture state measuring device 38, and balance measuring device 39) disposed in the wellness center CA. Each of the passive exercise machine 1 and the health state measurement unit 4 is provided with means for acquiring the user ID of the user and means for performing communication with the server 60 via a LAN.

The server 60 disposed in the community C is provided with a registered machine table TB21, a user attribution table TB22, a data table TB23, a PT table TB 24, an insurance company table TB25, and a community table TB26. The registered machine table TB21 stores therein machine ID allocated to machines in the community C, arrangement location thereof, and machine IP addresses which are registered in advance. The attribution table TB22 stores therein attribution information (user ID, password, gender, age, location, phone number, fax number, Email address, insurance policy number, and the like) of each individual user which are registered in advance. The data table TB23 stores therein history information (user ID, machine operation time interval (exercise time interval), and the like) of the exercise performed by each user, information (height, weight, blood pressure, pulse rate, amount of activity, visceral fat surface area, posture information) measured by the health state measurement unit 4, exercise points, health promotion points, health diagnostic points, and interview answers. The PT table TB 24 stores therein information (PT_IP allocated to individual physical therapist and an IP address (PT_ID address) thereof) on the physical therapist. The insurance company table TB25 stores therein information on the individual insurance company H (insurance company ID allocated to the individual insurance company H and IP address (insurance company IP address) thereof). The community table TB26 stores therein information on the community C (community ID allocated to the individual community C and IP address (community IP address) thereof).

Further, the server 60 is provided with a wellness center machine communication means (referred to hereinbelow as communication means) 61, a home machine communication means (referred to hereinbelow as communication means) 62, an external communication means 63, a machine reception data reading means 64, a machine verification means 65, a user verification means 66, user data storage means 67, user data extraction means 68, a the transmission data creation means 69, a transmission destination selection means 70, a PT - insurance company verification means 71, an external reception data reading means 72, a user point diagnostic report storage means 73, and a search means 74. The communication means 61 performs communication with the passive exercise machine 1 and health state measurement unit 4 in the wellness center CA via a LAN. The communication means 62 performs communication with the passive exercise machine 1 in the home CB via a LAN. The external communication means 63 performs data communication with the center server 2 via a WAN. The machine reception data reading means 64 reads the user ID, machine ID, machine operation time interval, or various measurement data from the data received by the two communication means 61, 62. The machine verification means 65 performs verification processing of whether the machine is a registered machine on the basis of the machine ID received by the communication means 61, 62 by referring to the registered machine table TB21. The user verification means 66 performs user verification on the basis of the received user ID by referring to the user attribution table TB22 when the machine verification is successful. The user data storage means 67 stores therein the data read by the machine reception data reading means 64 in the data table TB23 for each user separately when the user verification is successful. The user data extraction means 68 extracts data of a specific user from the data table TB23. The transmission data creation means 69 creates transmission data for transmitting the data extracted by the user data extraction means 68 to the PT_IP address input from the transmission destination selection means 70 and then transmits the created data to the external communication means 63. The transmission destination selection means 70 reads a PT_IP address of the desired physical therapist from the PT table TB24 on the basis of the input from the transmission destination input means 77 and then outputs the read-out address to the transmission data creation means 69. The PT - insurance company verification means 71 performs the verification with reference to the PT_ID and insurance company ID registered in the PT table TB24 or insurance company table when a verification request is received by the external communication means 63 from the servers 8, 9 of physical therapist G or insurance company H. The external reception data reading means 72 reads data on a predetermined item from the data received by the external communication means 63 when the verification performed by PT - insurance company verification means 71 is successful. The user point diagnostic report storage means 73 stores therein the data read by the external reception data reading means 72 in the data table TB23 for each user separately. The search means 74 searches the data in the data table TB23 on the basis of the search conditions input by the search condition input means 75 and displaying the search result on display means 76. When the communication destination of the external communication means 63 is the server 8 of the physical therapist G, the transmission data includes the community ID, user ID, machine operation time interval and various measurement data measured by the health state measurement unit 4. The reception data includes the community ID, user ID, exercise points, health promotion points, interview data, and health diagnostic report. When the communication destination of the external communication means 63 is the server 9 of the insurance company H, the community ID, user ID, insurance policy number, exercise points, and health promotion points are included in the transmission data, and the community ID, user ID insurance policy number, and insurance points are included in the reception data.

In the example shown in FIG. 11, the server 60 provided in the community C performs data communication between the passive exercise machine 1 and the health state measurement unit 4 disposed in the wellness center CA or home CB. Then, the server 60 acquires the operation time interval of the passive exercise machine 1 and various measurement data on health assessment indexes obtained with the health state measurement unit 4, transmitting these data to the center server 2, and exchanging data between the server 8 used by the physical therapist G and the server 9 of the insurance company H.

In the above-described embodiments, an example is explained in which health of AARC residents is promoted by using the exercise machine system in accordance with the present invention in the AARC. The exercise machine system in accordance with the present invention may be also used to obtain the same effect in communities in which elderly people live and are engaged in common activities, such as assisted living homes and nursing homes.

As described above, it is obvious that widely different embodiments can be made without departing from the spirit and scope of the present invention, and the present invention is defined by the annexed claims and not limited to the specific embodiments.

## Claims

1. An exercise administration system that is an exercise machine system, in which one or a plurality of exercise machines for allowing a user to perform an exercise is connected to a server that is operated by a service provider of a service that promotes health of the user via a network, wherein
the exercise machine comprises: an exercise load application means for applying an exercise load to the user; an operation control means for changing an operation state of the exercise load application means in response to a machine control signal input from the server; an identifier storage means for storing user identifiers that have been allocated to individual users; an operation time interval measurement means for measuring an operation time interval of the exercise machine; a measurement data storage means for storing measurement results of the operation time interval in association with the user identifiers; and a communication means for communicating with the server via the network, and
the server comprises: a communication means for communicating with the exercise machine via the network; a server storage means for storing the user identifiers and the measurement data on the operation time interval that have been received by the communication means from the exercise machine in association with each other; a point conversion means for determining exercise points obtained by quantifying an exercise amount of each user from the operation time interval of the user stored in the server storage means and storing the exercise points in the server storage means; and a machine operation control means for transmitting, from the communication means to the exercise machine, a machine control signal for changing an operation state of the exercise machine for the user when the cumulative total number of exercise points for each user that has been stored in the server storage means exceeds a predetermined threshold.

2. The exercise administration system according to claim 1, wherein the user is a resident of a community that provides to the residents thereof a living environment enabling the residents to lead an active life, and
the server is provided with means for transmitting exercise points of each user that have been read from the server storage means to at least either of an administrator of the community and the user.

3. The exercise administration system according to claim 1, wherein the user is a resident of a community that provides to the residents thereof a living environment enabling the residents to lead an active life, and
the server is provided with an exercise point totaling means for reading exercise points of all of the residents living in the community from the server storage means and totaling the exercise points, and a means for transmitting, upon receiving a request from the service provider that operates the server, a cumulative total number of exercise points to an advertiser that provides an advertisement for new residents to the community on the Internet, thereby updating the cumulative total number of exercise points displayed on the advertisement for new residents to the community.

4. The exercise administration system according to claim 1, wherein the user is a resident of a community that provides to the residents thereof a living environment enabling the residents to lead an active life, and a computer used by a physical therapist is connected to the network,
the exercise administration system comprises health state measurement means for measuring a health assessment index for evaluating a health state of the user,
the server is provided with a means for storing measurement data on the health assessment indexes obtained with the health state measurement means in association with the user identifiers in the measurement data storage means when the measurement data on the health assessment indexes are received by the communication means, and a means for reading the operation time interval and measurement data on the health assessment index for each user from the measurement data storage means and transmitting the read-out data from the communication means to the computer used by the physical therapist, and
upon receiving a response with diagnostic results from the computer used by the physical therapist, the communication means of the server stores the diagnostic results in association with the user identifiers in the measurement data storage means, and the communication means transmits the diagnostic results to at least one of the administrator of the community, the user, and a family member of the user.

5. The exercise administration system according to claim 4, wherein the user is a resident of a community that provides to the residents thereof a living environment enabling the residents to lead an active life, and a computer used by a physical therapist and a computer of an insurance company that has signed a medical insurance contract with the residents are connected to the network;
the server is provided with means for reading diagnostic results of the users diagnosed by the physical therapist from the measurement data storage means and transmit the diagnostic results from the communication means to the computer of the insurance company, and
the computer of the insurance company is provided with means for receiving the diagnostic results of the users transmitted from the server, and means for calculating a reduction ratio of insurance premium on the basis of the diagnostic results of the users, and the insurance premium of the medical insurance is set on the basis of the diagnostic result obtained by the physical therapist.
